# FASCICULE DE BREVET EUROPEEN

(11) **EP 1 467 962 B1**
(45) Date de publication et mention de la délivrance du brevet: **12.11.2008**
(21) Numéro de dépôt: 03717344.0
(22) Date de dépôt: 20.01.2003
(51) Int. Cl.: C07C 209/48, C07C 253/30

(54) **PROCEDE CONTINU D'HYDROGENATION DE NITRILES OU COMPOSES NITRES EN AMINES**
KONTINUIERLICHES HYDRIERVERFAHREN VON NITRILEN ODER NITROVERBINDUNGEN ZU AMINEN
CONTINUOUS METHOD FOR HYDROGENATION OF NITRILES OR NITRATED COMPOUNDS TO GIVE AMINES

(30) Priorité: 21.01.2002 FR 0200703
(43) Date de publication de la demande: 20.10.2004
(73) Titulaire: RHODIA POLYAMIDE INTERMEDIATES, 69192 Saint-Fons (FR)
(72) Inventeur: BOCQUENET, Gérald, F-69360 Communay (FR); CHESNAIS, André, F-69360 Saint-Symphorien d'Ozon (FR); DESIRE, Jean-Michel, F-69100 Villeurbanne (FR); LECONTE, Philippe, F-69330 Meyzieu (FR); SEVER, Lionel, Tardy et Rozon, F-38200 Luzinay (FR)
(74) Mandataire: Esson, Jean-Pierre
(86) Numéro de dépôt international: PCT/FR2003/000161
(87) Numéro de publication internationale: WO 2003/062188

(56) Documents cités:
- FR-A- 2 749 191
- FR-A- 2 773 086
- US-B1- 6 232 488

## Description

La présente invention concerne un procédé d'hydrogénation de composés comprenant des fonctions nitriles ou nitrés en composés amines, aminonitriles ou aminonitrés.

Elle se rapporte plus particulièrement à un procédé continu d'hydrogénation.

L'hydrogénation des dinitriles en diamines correspondantes est un procédé utilisé depuis longtemps, notamment l'hydrogénation de l'adiponitrile en hexaméthylènediamine, l'une des matières de base de la préparation du polyamide 66.

Depuis quelques années est apparu un intérêt croissant à l'hydrogénation (aussi parfois appelée hémihydrogénation) des dinitriles aliphatiques en aminonitriles, notamment l'hydrogénation de l'adiponitrile en 6-aminocapronitrile conduisant soit directement, soit par l'intermédiaire du caprolactame, au polyamide 6.

Ainsi le brevet US-A-5 151 543 décrit un procédé d'hydrogénation sélective de dinitriles aliphatiques en aminonitriles correspondants, à 25-150°C et sous pression supérieure à la pression atmosphérique, en présence d'un solvant en excès molaire d'au moins 2/1 par rapport au dinitrile, le solvant contenant de l'ammoniac liquide ou un alcool de 1 à 4 atomes de carbone et une base minérale soluble dans ledit alcool, en présence d'un catalyseur Raney, l'aminonitrile obtenu étant récupéré comme produit principal.

Le brevet WO-A-93/16034 décrit un procédé de préparation de 6-aminocapronitrile par hydrogénation de l'adiponitrile, en présence d'une base minérale, d'un complexe de métal de transition de faible valence choisi parmi le chrome, le tungstène, le cobalt et le fer et de nickel de Raney comme catalyseur, sous pression d'hydrogène et à une température de 50°C à 90°C.

Le brevet WO-A-96/18603 décrit l'hémihydrogénation de dinitriles aliphatiques en aminonitriles par l'hydrogène et en présence d'un catalyseur à base de nickel ou de cobalt de Raney éventuellement dopé et d'une base minérale forte, le milieu initial d'hydrogénation comportant de l'eau, de l'aminonitrile et/ou de la diamine susceptibles de se former et du dinitrile non transformé.

Tous ces procédés d'hydrogénation conduisent à l'aminonitrile et sont présentés comme pouvant être mis en oeuvre en continu dans une installation industrielle.

Toutefois, pour obtenir une économie du procédé compatible avec une exploitation industrielle, il est nécessaire de pouvoir recycler le réactif non transformé et avoir une consommation de catalyseur par kilogramme de composés produits, la plus faible possible.

Il a déjà été proposé dans le brevet français 2 749 191 un procédé permettant de prélever une partie des produits hydrogénés à partir du milieu réactionnel sans prélever de catalyseur par mise en oeuvre d'une filtration tangentielle en présence d'hydrogène gazeux pour éviter une désactivation du catalyseur.

Toutefois, ce document ne décrit pas un procédé permettant également une régénération du catalyseur en continu pour permettre un fonctionnement totalement continu du procédé.

Un des buts de la présente invention est de proposer un procédé d'hydrogénation permettant un fonctionnement en continu avec recyclage et régénération du catalyseur. Un tel procédé permet de maintenir une sélectivité et un rendement élevé de l'hydrogénation et une mise en oeuvre à l'échelle industrielle de celui-ci.

A cet effet, l'invention propose un procédé continu d'hydrogénation de composés comprenant des fonctions nitriles ou nitro en composés amine ou aminonitriles en présence d'un catalyseur d'hydrogénation hétérogène sous forme divisée et d'un composé basique caractérisé en ce qu'il consiste à :
- alimenter dans un réacteur agité :
   . un premier flux de réactif à hydrogéner,
   . un second flux de catalyseur,
   . un troisième flux de composé basique,
   . un quatrième flux d'hydrogène pour maintenir le réacteur sous pression d'hydrogène.
- soutirer du réacteur au moins un cinquième flux constitué par le milieu réactionnel, et comprenant des bulles d'hydrogène dispersées dans ledit milieu,
- faire circuler ce cinquième flux dans au moins une boucle débouchant à la partie inférieure et à la partie supérieure du réacteur et éliminer par échange de chaleur avec ledit cinquième flux, les calories produites par la réaction d'hydrogénation de manière à maintenir le milieu réactionnel à une température inférieure à 150°C,
- prélever à partir de ce cinquième flux, circulant dans une des boucles, un sixième flux contenant une partie de l'hydrogénat séparé du catalyseur,
- prélever à partir du réacteur ou d'une des boucles de circulation, un septième flux d'hydrogénat qui est alimenté dans une étape de séparation liquide/solide et récupérer la phase liquide contenant l'hydrogénat exempt de catalyseur et la phase solide constituée par le catalyseur, ladite phase solide étant traitée pour régénération avant d'être recyclée dans le second flux de catalyseur.

Le procédé de l'invention comprend une circulation du milieu réactionnel à l'extérieur du réacteur permettant d'une part d'évacuer de manière contrôlée et simple les calories générées par la réaction, et d'autre part de soutirer l'hydrogénat sans perturbation de la concentration en catalyseur dans le milieu réactionnel et sans provoquer sa désactivation. Enfin, le procédé de l'invention permet un remplacement en continu du catalyseur par un mélange de catalyseur neuf et de catalyseur régénéré.

Par "hydrogénat" on désigne dans le présent texte la phase liquide du milieu réactionnel sortant du réacteur. Cet hydrogénat comprend les composés hydrogénés et les réactifs qui n'ont pas été transformés, ainsi que les solvants si ils sont présents.

Selon une caractéristique avantageuse de l'invention, un échangeur de chaleur est présent dans une des boucles de circulation du milieu réactionnel. Quand cet échangeur de chaleur est disposé sur la boucle comprenant le soutirage du sixième flux d'hydrogénat appelé pour plus de clarté première boucle de circulation, cet échangeur est situé en aval dudit soutirage du sixième flux d'hydrogénat par rapport au sens de circulation du milieu réactionnel. Cet échange de chaleur permet d'évacuer les calories générées par la réaction d'hydrogénation et de maintenir le milieu réactionnel à une température déterminée. Ce contrôle de la température est important car il permet d'obtenir une sélectivité désirée notamment dans le cas d'une hydrogénation partielle de dinitriles en aminonitriles.

Ce contrôle de la température ou évacuation des calories peut être réalisé, dans une seconde variante du procédé de l'invention, par disposition d'un échangeur de chaleur sur une seconde boucle de circulation du milieu réactionnel dans laquelle circule un huitième flux dudit milieu réactionnel soutiré en partie inférieure du réacteur et recyclé après refroidissement dans ledit réacteur. Dans cette variante, la première boucle de circulation ne comprend avantageusement pas d'échangeur de chaleur permettant ainsi de réduire globalement le temps de séjour du milieu réactionnel dans chaque boucle de circulation et ainsi limiter la désactivation du catalyseur. En effet, le catalyseur contenu dans un milieu contenant des composés nitriles avec une concentration en hydrogène faible, se désactive rapidement. Comme la réaction d'hydrogénation continue dans la boucle de circulation, la concentration en hydrogène diminue rapidement. Un avantage du procédé de l'invention est de permettre une durée très courte de circulation du milieu réactionnel dans les boucles, donc de limiter au minimum le temps de séjour du catalyseur et de maintenir dans l'ensemble des boucles de circulation du milieu réactionnel un milieu triphasique comprenant le catalyseur sous forme solide, l'hydrogénat sous forme liquide et des bulles d'hydrogène dispersées.

Le prélèvement ou le soutirage de l'hydrogénat est réalisé, selon le procédé de l'invention sur la première boucle de circulation du milieu réactionnel. Ce prélèvement est effectué à travers un milieu filtrant pour ainsi maintenir la totalité du catalyseur dans le milieu réactionnel en circulation. Comme milieu filtrant, on peut utiliser tout type de matériau résistant à la pression du milieu réactionnel et aux conditions de température et d'agressions chimiques.

Dans un mode de réalisation préféré et pour permettre un prélèvement sans augmenter le temps de séjour du milieu réactionnel dans la boucle de circulation, ce prélèvement est effectué avantageusement avec une filtration de type tangentielle. Le milieu filtrant est avantageusement constitué par un média poreux disposé tangentiellement au sens de circulation du cinquième flux dans la première boucle de circulation. Un exemple de filtration tangentielle convenable pour l'invention est celle décrite dans le brevet français n°2749191.

Comme médias poreux convenables pour l'invention on peut citer les médias frittés métalliques, en carbone ou des médias constitués par une membrane minérale ou organique, disposée sur un support plan ou tubulaire. Ladite membrane appelée couche active, présente une épaisseur de quelques micromètres d'épaisseur, par exemple comprise entre 5 µm et 100 µm.

Les médias métalliques présentant une meilleure tenue vis à vis des produits chimiques et thermiques sont préférés. A titre d'exemple, ces médias sont par exemple réalisés en acier inoxydable.

Par ailleurs, ces médias poreux peuvent être facilement entretenus et nettoyés. Ainsi, il est possible de régénérer le milieu filtrant après une certaine durée d'utilisation par lavage avec différentes solutions, comme l'eau, les acides, les bases.

De préférence, le prélèvement d'hydrogénat représente de 0,1 % à 10 % en volume du débit de milieu réactionnel circulant dans la première boucle.

Avantageusement, pour éviter l'entraînement de catalyseur dans le sixième flux d'hydrogénat, le média poreux présente des pores de diamètre compris entre 1 nanomètre et 10 micromètres.

Selon une caractéristique préférentielle, notamment dans le cas d'une hydrogénation partielle des composés en produits aminonitriles ou aminonitrés, l'étape de filtration est réalisée en présence d'hydrogène sous forme gaz, le milieu réactionnel étant saturé en hydrogène dissous. Cet hydrogène gazeux peut provenir, avantageusement, des bulles de gaz dispersées dans le milieu réactionnel par l'agitation présente dans le réacteur. Il est également possible de prévoir une alimentation d'hydrogène dans les boucles de circulation pour maintenir une présence d'hydrogène sous forme gaz dans l'ensemble des boucles.

Le procédé de l'invention permet donc de soutirer l'hydrogénat représentant la production du procédé sans extraire du catalyseur du milieu réactionnel et surtout dans des conditions évitant la désactivation de celui-ci. Cet aspect est notamment important quand le milieu réactionnel contient des composés avec des fonctions nitriles ou nitrées non encore hydrogénées.

Selon une autre caractéristique de l'invention, le procédé comprend une étape permettant de soutirer une partie du catalyseur pour permettre son traitement pour une régénération avant son recyclage dans le procédé d'hydrogénation. Ainsi, soit sur une des boucles de circulation du milieu réactionnel ou soit directement par soutirage à partir du réacteur, une partie du milieu réactionnel est prélevée de manière continue ou périodiquement pour former un septième flux. Ce septième flux comprenant de l'hydrogénat et du catalyseur est traité dans une étape de séparation solide/liquide. Tout procédé de séparation liquide/solide peut être utilisé, tel que filtration, centrifugation, décantation. Le catalyseur ainsi séparé est avantageusement lavé par de l'eau, de préférence légèrement basique (pH avantageusement supérieur à 9), pour récupérer l'hydrogénat. Les eaux de lavage sont ainsi, avantageusement ajoutées dans la phase liquide séparée. Ce mélange représente également une production d'hydrogénat et peut être ajouté au sixième flux récupéré dans l'étape de filtration tangentielle.

L'hydrogénat peut être utilisé tel quel dans d'autres procédés de synthèse ou purifié, par exemple, par distillation. Ainsi, les différents produits contenus dans l'hydrogénat peuvent être séparés par distillation dans plusieurs colonnes montées en série.

Le catalyseur récupéré est avantageusement soumis à un traitement de régénération avant d'être recyclé dans le second flux de catalyseur alimenté dans le réacteur.

La régénération du catalyseur est effectuée selon des procédés connus, notamment par le procédé décrit dans la demande de brevet FR 2773086. Le procédé consiste, dans le cas de catalyseur de type de Raney, soit à traiter le catalyseur avec une solution basique pour dissoudre au moins une partie des composés aluminates formés soit à hydrogéner le catalyseur en milieu basique. De manière générale, les procédés de régénération comprennent une étape de lavage pour éliminer les composés accumulés sur le catalyseur et éventuellement une étape de réduction, par exemple, par l'hydrogène.

Comme indiqué précédemment, la récupération du catalyseur peut être effectuée indifféremment sur une des boucles de circulation du milieu réactionnel ou par soutirage directement à partir du réacteur.

Le procédé de l'invention permet donc simultanément de récupérer l'hydrogénat et de recycler du catalyseur régénéré sans perturbation du fonctionnement du réacteur et sans induire ou générer une désactivation rapide du catalyseur présent dans le milieu réactionnel.

Le procédé de l'invention s'applique à l'hydrogénation totale ou partielle de composés comprenant des fonctions nitriles ou nitrés. Il s'applique plus particulièrement à un procédé d'hydrogénation partielle ou hémihydrogénation de composés comprenant des fonctions nitriles ou polynitriles.

Ce procédé est avantageusement utilisé pour l'hydrogénation d'adiponitrile en aminocapronitrile et hexaméthylène diamine. L'aminocapronitrile peut être utilisé pour la fabrication d'ε-caprolactame ou dans un procédé de polymérisation pour la fabrication de polyamides.

Les composés nitriles convenables pour l'invention sont notamment les composés dinitriles aliphatiques.

Ainsi, Les dinitriles aliphatiques qui peuvent être mis en oeuvre dans le procédé de l'invention sont plus particulièrement les dinitriles de formule générale (I) :

NC-R-CN (I)

dans laquelle R représente un groupement alkylène ou alcénylène, linéaire ou ramifié, ayant de 1 à 12 atomes de carbone.

De préférence, on met en oeuvre dans le procédé de l'invention des dinitriles de formule (I) dans laquelle R représente un radical alkylène, linéaire ou ramifié ayant de 2 à 6 atomes de carbone.

A titre d'exemple de tels dinitriles, on peut citer notamment l'adiponitrile, le méthylglutaronitrile, l'éthylsuccinonitrile, le malononitrile, le succinonitrile, le glutaronitrile et leurs mélanges, notamment les mélanges d'adiponitrile et/ou de méthylglutaronitrile et/ou d'éthylsuccinonitrile susceptibles de provenir d'un même procédé de synthèse de l'adiponitrile.

En pratique, le cas où R = (CH₂)₄ sera le plus fréquent car cela correspond à la mise en oeuvre de l'adiponitrile (AdN) dans le présent procédé.

Selon une variante particulièrement avantageuse de la présente invention, les composés à hydrogéner sont des composés aromatiques comprenant au moins une fonction nitro.

De préférence, lesdits composés présentent au moins deux fonctions nitro, et au moins un motif aromatique en C₆-C₁₄, de préférence en C₆-C₁₀, substitué ou non par un ou plusieurs radicaux hydrocarbonés, saturés ou non, linéaires, cycliques ou ramifiés en C₁-C₁₀, et/ou un ou plusieurs radicaux hydroxyles.

Plus précisément, les radicaux hydrocarbonés précités, substituant éventuellement lesdits motifs aromatiques, peuvent être choisis parmi les radicaux alkyles, aryles, alkylaryles et arylalkyles en C₁-C₁₀, de préférence en C₁-C₆.

Comme motif aromatique, on peut notamment citer les noyaux benzénique et naphtalénique, substitués ou non par un ou plusieurs radicaux méthyle, éthyle, propyle, butyle, pentyle, hexyle et/ou leurs isomères.

Le procédé selon l'invention peut être mis en oeuvre en utilisant au moins un composé parmi le mononitrobenzène, le mononitrotoluène, le dinitrotoluène, le paranitrocumène, l'orthonitrophénol.

Dans le cas d'hydrogénation de composés dinitriles, la composition moyenne du milieu réactionnel est déterminée en fonction des taux de conversion et de sélectivité désirés.

Ainsi, le milieu réactionnel comprend avantageusement une quantité d'eau inférieure ou égale à 20%. Préférentiellement, la teneur en eau est comprise entre 1,5 % et 7 % en poids par rapport à l'ensemble des constituants liquides du milieu.

Toutefois, ce procédé peut être réalisé en absence d'eau mais en présence soit d'ammoniac liquide soit d'alcools aliphatiques.

Généralement, le procédé d'hydrogénation est mis en oeuvre en présence d'un solvant qui peut correspondre à un des produits obtenus par hydrogénation. Ainsi, dans le cas de l'hydrogénation de l'adiponitrile, le solvant est avantageusement de l'hexaméthylène diamine.

Les catalyseurs utilisés dans ce procédé d'hémihydrogénation peuvent être un nickel de Raney, un cobalt de Raney, comportant, outre le nickel ou le cobalt et les quantités résiduelles du métal éliminé de l'alliage d'origine lors de la préparation du catalyseur, c'est-à-dire généralement l'aluminium, un ou plusieurs autres éléments, souvent appelés dopants, tels que par exemple le chrome, le titane, le molybdène, le cuivre, le tungstène, le fer, le zinc, le rhodium, l'iridium. Parmi ces éléments dopants le chrome, le cuivre, le titane, le fer, le rhodium, l'iridium et leurs mélanges sont considérés comme les plus avantageux. Ces dopants représentent habituellement, en poids par rapport au poids de nickel ou de cobalt, de 0 % à 15 % et de préférence de 0 % à 10 %.

On peut également utiliser, avantageusement un catalyseur à base de ruthénium ou de rhodium déposé sur un support. Ce catalyseur peut également comprendre des éléments métalliques dopants compris dans la liste citée pour les métaux de Raney.

La quantité de catalyseur mise en oeuvre peut varier très largement en fonction notamment de la nature du catalyseur ou des conditions réactionnelles choisies. A titre indicatif, on peut utiliser de 0,5 % à 20 % en poids de catalyseur par rapport au poids total du milieu réactionnel et le plus souvent de 1 % à 12 % en poids.

Le deuxième flux de catalyseur comprenant le catalyseur régénéré et le catalyseur neuf est avantageusement conditionné avant d'être introduit dans le réacteur d'hydrogénation. Ce conditionnement est notamment décrit dans le brevet français 2806081. Ce procédé consiste à ajouter le catalyseur dans un solvant puis à additionner dans ce mélange la quantité de base nécessaire pour conditionner le catalyseur. Le solvant est choisi pour être un mauvais solvant du composé basique permettant ainsi une fixation, par exemple par adsorption, des molécules de composés basiques, généralement des bases minérales fortes, à la surface du catalyseur.

Les bases minérales fortes convenables pour l'invention sont les hydroxydes de métaux alcalins ou alcalino-terreux, par exemple LiOH, NaOH, KOH, RbOH, CsOH, les hydroxydes de tétraalkyle d'ammonium comme l'hydroxyde de tétraéthyl ou méthyl d'ammonium et leurs mélanges.

Le procédé d'hydrogénation de l'invention est généralement mis en oeuvre à une température réactionnelle inférieure ou égale à 150°C, de préférence inférieure ou égale à 120°C et, plus préférentiellement encore, inférieure ou égale à 100°C.

Concrètement, cette température est comprise entre la température ambiante (20°C environ) et 100°C.

Préalablement, simultanément ou postérieurement au chauffage, l'enceinte réactionnelle est amenée à la pression en hydrogène convenable, c'est-à-dire, en pratique, comprise entre 1bar (0,10 MPa) et 100 bar (10 MPa) et de préférence entre 5 bar (0,5 MPa) et 50 bar (5 MPa).

Les autres conditions qui régissent le procédé d'hydrogénation conforme à l'invention, relèvent de dispositions techniques traditionnelles et connues en elles-mêmes.

Par ailleurs, ces conditions peuvent être modifiées pour adapter le taux de transformation du dinitrile selon qu'il est souhaité une forte sélectivité en aminonitrile ou inversement une hydrogénation totale des dinitriles en diamines.

Selon une autre caractéristique préférée de l'invention, le milieu liquide de conservation du métal de Raney est, de préférence, l'eau. La concentration en aluminium dans le métal de Raney est de préférence comprise entre 2 et 6 %. Un faible taux d'aluminium dans le catalyseur est préférable car il permet de faire varier la sélectivité de la réaction et de diminuer le phénomène de colmatage des médias poreux.

Selon une caractéristique de l'invention, la quantité de base forte ajoutée dans l'étape de conditionnement du catalyseur est comprise entre 0,1 mol et 50 mol par kg de catalyseur. La quantité optimale de base est déterminée pour chaque catalyseur.

Selon un mode préféré de l'invention, la base forte est ajoutée dans l'étape de conditionnement sous forme de solution concentrée ou sous forme pure.

Par ailleurs, la quantité de solvant ajouté dépend du degré de solubilité de l'eau dans ce solvant et du niveau de concentration désiré dans la phase contenant la base forte. Avantageusement, le rapport en poids entre le solvant et l'eau sera au moins égal à 1, de préférence supérieur ou égal à 2.

Selon l'invention, le solvant est choisi parmi les composés qui ont une affinité (pouvoir de solubilisation par exemple) avec l'eau ou le liquide de conservation du métal de Raney et qui, au contraire, n'ont pas d'affinité (pouvoir de solubilisation faible) avec la base minérale forte. Par insolubilité de la base forte dans le solvant ou plus précisément dans la phase liquide formée par le solvant et l'eau ou le liquide de conservation, il faut comprendre une solubilité faible de la base, par exemple inférieure à 1 % en poids.

Dans un mode de réalisation préféré de l'invention, le solvant est avantageusement une amine, de préférence une amine correspondant à celle obtenue par la réaction d'hydrogénation ou l'ammoniac liquide dans le cas où l'hydrogénation serait conduite en milieu ammoniac liquide. En effet, le choix du solvant doit permettre, avantageusement, de ne pas introduire de nouveaux produits dans le milieu d'hydrogénation et donc de permettre des procédés de séparation et éventuellement de recyclage aisés et peu coûteux donc peu pénalisant d'un point de vue technique et économique pour le procédé.

Une quantité supplémentaire de composé basique peut être ajoutée dans le milieu réactionnel sous forme d'un flux séparé ou en mélange avec le flux d'eau ou de solvant. Cette quantité supplémentaire de base est destinée à saturer le milieu réactionnel en composés basiques pour ne pas modifier la quantité de base déposée sur le catalyseur.

D'autres détails, avantages de l'invention apparaîtront plus clairement au vu de la description détaillée d'un mode de réalisation de l'invention donné uniquement à titre illustratif et faite en référence aux figures en annexe dans lesquelles :

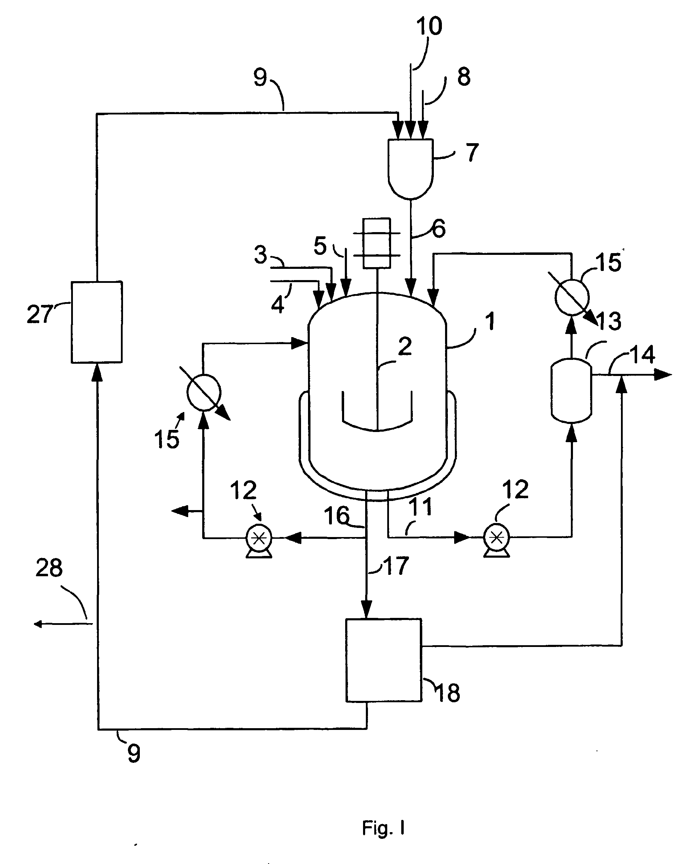

- la figure 1 représente un schéma synoptique du procédé de l'invention, et
- la figure 2 représente un schéma synoptique du dispositif de séparation solide / liquide.

Le procédé de l'invention est réalisé dans un réacteur 1 d'hydrogénation comprenant un agitateur 2 permettant de maintenir en suspension le solide dans le milieu réactionnel ainsi que de disperser le gaz (l'hydrogène) formant le ciel du réacteur sous forme de bulles. L'agitateur peut être, par exemple, un agitateur autoaspirant de type à cavitation (type "cavitator"), de type Rushton ou de type multipales, avantageusement inclinées.

Cette agitation permet d'obtenir un milieu triphasique comprenant une phase solide constituée par le catalyseur, une phase liquide constituée principalement par les différents composés organiques à hydrogéner ou hydrogénés et une phase gaz constituée par les bulles d'hydrogène dispersées dans le milieu réactionnel.

Le réacteur peut comprendre une double enveloppe pour permettre le contrôle de la température par utilisation d'un fluide caloporteur.

Le réacteur 1 comprend un premier conduit 3 d'alimentation du réactif à hydrogéner, formant le premier flux. Dans l'exemple illustré, ce réactif est de l'adiponitrile, avantageusement en solution dans un solvant tel que l'hexaméthylène diamine. Il comprend un second conduit 4 d'alimentation de composés basiques avantageusement en solution aqueuse, formant le troisième flux, par exemple une solution aqueuse de potasse.

Dans le mode de réalisation illustré, l'hydrogénation est réalisée en présence d'une quantité d'eau comprise entre 1,5 % et 7 % en poids de la masse liquide du milieu réactionnel, cette eau étant avantageusement alimentée sous forme de solvant des composés basiques. Toutefois, le procédé de l'invention peut être réalisé en absence d'eau et en présence d'autres solvants tels que l'ammoniac, les alcools aliphatiques comme le méthanol, l'éthanol ou le propanol. Dans ce dernier cas, les composés basiques sont ajoutés sous forme de solutions alcooliques.

Le réacteur 1 comprend également un conduit 5 d'alimentation en gaz hydrogène constituant le quatrième flux et formant le ciel du réacteur.

Le catalyseur est introduit dans le réacteur par le conduit 6 sous forme d'une suspension dans un solvant préparée dans un récipient 7 de conditionnement. Cette suspension forme le deuxième flux.

Dans le mode de réalisation illustré, cette suspension de catalyseur est obtenue par introduction dans le récipient 7, par le conduit 8 d'un solvant comme l'hexaméthylène diamine, et par le conduit 9 du catalyseur recyclé après éventuellement régénération. Le catalyseur neuf et une solution de composés basiques, par exemple une solution aqueuse de potasse, sont également introduits par le conduit 10.

Selon l'invention, le milieu réactionnel est soutiré à la partie inférieure du réacteur 1 par un conduit 11 formant une première boucle de circulation, et réalimenté dans la partie supérieure du réacteur 1. Cette boucle de circulation comprend une pompe 12 compatible pour mettre en circulation un milieu triphasique. Le milieu réactionnel est alimenté dans un filtre tangentiel 13 représenté schématiquement. Dans ce filtre 13, une partie de la phase liquide est filtrée à travers le média poreux formé par une grille métallique en acier oxydable de diamètre de pore inférieur à 10 micromètres et soutirée par le conduit 14.

Dans le mode de réalisation illustré, la boucle 11 comprend également un échangeur de chaleur 15 disposé après le filtre tangentiel 13 permettant de refroidir le milieu réactionnel à une température déterminée pour conserver une température dans le réacteur comprise dans un domaine désiré cité ci-dessus.

Le réacteur 1 comprend à sa partie inférieure, un second soutirage 16 du milieu réactionnel formant une seconde boucle de circulation. Une partie du milieu réactionnel circulant dans cette boucle 16 est prélevée par le conduit 17 et alimentée dans un dispositif 18 de séparation solide liquide. Cette seconde boucle de circulation peut également comprendre une pompe 12 et un échangeur de chaleur 15.

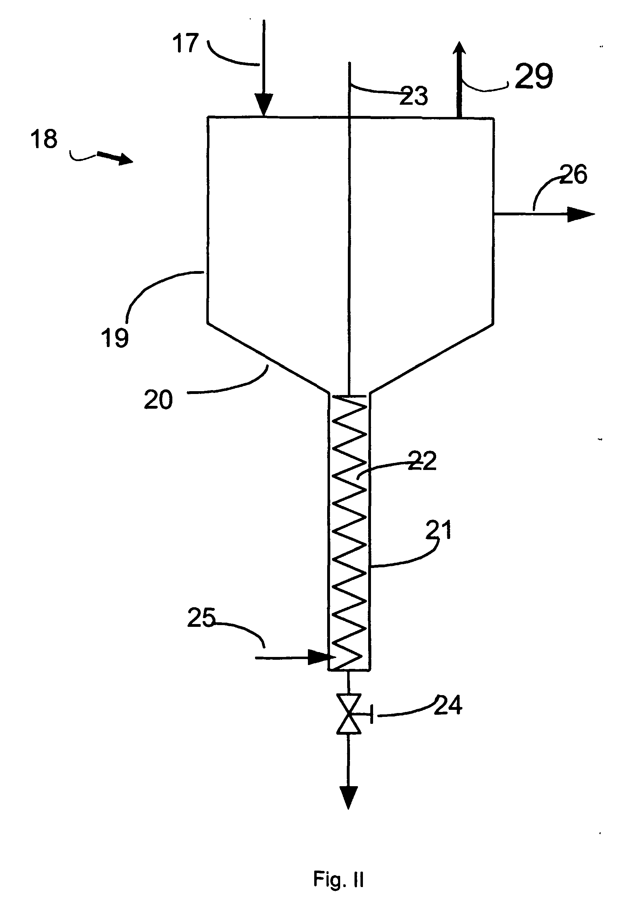

Un mode de réalisation particulier du dispositif 18 est illustré à la figure 2.

Ce dispositif 18 comprend une première partie 19 comprenant une partie inférieure 20 de forme conique. L'extrémité du cône 20 est prolongée par un conduit 21 de faible section dans lequel est disposé un moyen 22 pour maintenir en mouvement le solide qui s'accumule dans ledit conduit. Ce moyen 22 est, par exemple, une vis sans fin entraînée en mouvement par l'arbre 23 et un moteur non illustré. Le conduit 21 comprend à son extrémité inférieure une vanne 24 pour permettre le soutirage du solide présent. Un conduit 25 débouche dans le conduit 21, avantageusement à sa partie inférieure, pour permettre une alimentation en eau légèrement basique pour laver le catalyseur présent dans ledit conduit 21. Le milieu réactionnel soutiré par le conduit 17 est alimenté dans la première partie 19 du dispositif 18 par l'intermédiaire d'une vanne (non illustrée) ou dans un récipient intermédiaire utilisé comme sas (également non illustrée), la phase liquide surnageante est soutirée par un conduit 26 soit comme production du procédé d'hydrogénation soit pour être mélangée avec l'hydrogénat soutiré par le conduit 14 à travers le filtre 13. Le dispositif comprend également une sortie 29 permettant l'évacuation des gaz provenant du milieu réactionnel.

Ce mode de réalisation décrit ci-dessus n'est donné qu'à titre indicatif. D'autres dispositifs de séparation liquide/solide peuvent être employés sans sortir du cadre de l'invention.

Selon le mode de réalisation illustré, le catalyseur récupéré en sortie du conduit 21 par la vanne 24 est recyclé par le conduit 9 dans le récipient 7 de conditionnement du catalyseur. Avantageusement, le catalyseur est régénéré avant son introduction dans le récipient 7 dans une installation illustrée schématiquement en 27. Cette installation peut comprendre un réacteur permettant de traiter le catalyseur par l'hydrogène en milieu basique. Cette installation peut également comprendre un laveur du catalyseur, par exemple par une solution basique.

Le conduit 9 comprend avantageusement un piquage 28 permettant d'éliminer une partie du catalyseur usé.

L'installation décrite ci-dessus n'a été donnée qu'à titre d'illustration. Ainsi, la disposition de l'échangeur de chaleur, du prélèvement du catalyseur, le nombre de boucles de circulation du milieu réactionnel peuvent être différents sans sortir du cadre de l'invention.

## Revendications

1. Procédé continu d'hydrogénation de composés comprenant des fonctions nitriles ou nitro en composés amines ou aminonitriles en présence d'un catalyseur d'hydrogénation hétérogène sous forme divisée et d'un composé basique **caractérisé en ce qu'**il consiste à
- alimenter dans un réacteur agité ;
. un premier flux de réactif à hydrogéner,
. un second flux de catalyseur,
. un troisième flux de composé basique,
. un quatrième flux d'hydrogène pour maintenir le réacteur sous pression d'hydrogène.
- soutirer du réacteur au moins un cinquième flux constitué par le milieu réactionnel, et comprenant des bulles d'hydrogène dispersées dans ledit milieu,
- faire circuler ce cinquième flux dans au moins une boucle débouchant à la partie inférieure et à la partie supérieure du réacteur et éliminer par échange de chaleur avec ledit cinquième flux, les calories produites par la réaction d'hydrogénation de manière à maintenir le milieu réactionnel à une température inférieure à 150°C,
- prélever à partir de ce cinquième flux, circulant dans une des boucles, un sixième flux contenant une partie de l'hydrogénat séparé du catalyseur,
- prélever à partir du réacteur ou d'une des boucles de circulation, un septième flux d'hydrogénat qui est alimenté dans une étape de séparation liquide/solide et récupérer la phase liquide contenant l'hydrogénat exempt de catalyseur et la phase solide constituée par le catalyseur, ladite phase solide étant traitée pour régénération avant d'être recyclée dans le second flux de catalyseur.

2. Procédé selon la revendication 1 **caractérisé en ce que** l'élimination des calories est assurée par un échangeur de chaleur disposé en aval dudit prélèvement du sixième flux de composés hydrogénés sur la boucle de circulation dudit cinquième flux.

3. Procédé selon la revendication 1 ou 2, **caractérisé en ce qu'**un huitième flux du milieu réactionnel est soutiré du réacteur et mis en circulation dans une seconde boucle avant d'être recyclé dans le réacteur, l'élimination des calories étant assurée par un échangeur de chaleur disposé sur ladite boucle de circulation du huitième flux.

4. Procédé selon l'une des revendications précédentes, **caractérisé en ce que** le prélèvement du sixième flux est réalisé à travers un milieu filtrant.

5. Procédé selon la revendication 4, **caractérisé en ce que** le milieu filtrant est une média poreux disposé tangentiellement à la direction du cinquième flux circulant dans la boucle.

6. Procédé selon la revendication 4 ou 5, **caractérisé en ce que** le média poreux est métallique.

7. Procédé selon la revendication 4 ou 5, **caractérisé en ce que** le milieu filtrant comprend une membrane disposé sur un support.

8. Procédé selon l'une des revendications précédentes, **caractérisé en ce que** le milieu réactionnel comprend un solvant.

9. Procédé selon l'une des revendications précédentes, **caractérisé en ce que** le milieu réactionnel comprend de l'eau, de l'ammoniac ou un alcool.

10. Procédé selon la revendication 9, **caractérisé en ce que** le milieu réactionnel comprend de 1,5% à 7% en poids d'eau par rapport à la masse liquide du milieu réactionnel.

11. Procédé selon l'une des revendications 1 à 10, **caractérisé en ce que** la séparation liquide/solide mise en oeuvre sur le septième flux est une étape de décantation, de filtration ou de centrifugation.

12. Procédé selon la revendication 11, **caractérisé en ce que** la séparation liquide /solide est mise en oeuvre dans un dispositif comprenant une partie supérieure présentant un fond en forme de cône, et un conduit de faible section se prolongeant à partir de l'extrémité du cône, un moyen de mise en mouvement du solide étant disposé dans le conduit de faible section, le milieu réactionnel étant alimenté dans ladite partie supérieure, la phase liquide séparée du solide étant prélevée à partir de ladite partie supérieure, le solide décanté étant récupéré à la partie inférieure du conduit de faible section.

13. Procédé selon la revendication 12, **caractérisé en ce que** de l'eau est introduite dans le conduit de faible section.

14. Procédé selon l'une des revendications précédentes, **caractérisé en ce que** la phase solide récupérée est soumise à une étape de régénération du catalyseur.

15. Procédé selon la revendication 14, **caractérisé en ce que** le catalyseur régénéré est alimenté dans le second flux de catalyseur.

16. Procédé selon l'une des revendications précédentes, **caractérisé en ce que** le second flux de catalyseur est constitué d'un mélange comprenant le catalyseur, un composé basique et éventuellement un solvant dans lequel le composé basique n'est pas soluble, le rapport composé basique/catalyseur étant compris entre 0,1 mol et 50 moles de composés basique pour 1 kg de catalyseur.

17. Procédé selon la revendication 16, **caractérisé en ce que** le catalyseur dans le second flux de catalyseur est un mélange de catalyseur neuf et de catalyseur régénéré.

18. Procédé selon l'une des revendications précédentes, **caractérisé en ce que** le catalyseur est un nickel de Raney, cobalt de Raney comprenant entre 2 % et 6 % du poids d'aluminium et éventuellement un dopant choisi parmi les métaux choisi dans le groupe, le chrome, le titane, le molybdène, le cuivre, le tungstène, le fer, le zinc, le rhodium, l'iridium.

19. Procédé selon l'une des revendications précédentes, **caractérisé en ce que** le composé polynitrile est l'adiponitrile.

## Claims

1. Continuous process for the hydrogenation of compounds comprising nitrile or nitro functional groups to amine or aminonitrile compounds in the presence of a heterogeneous hydrogenation catalyst in divided form and of a basic compound **characterized in that** it consists in:
- feeding, into a stirred reactor:
. a first stream, of reactant to be hydrogenated,
. a second stream, of catalyst,
. a third stream, of basic compound and
. a fourth stream, of hydrogen in order to maintain the reactor under a pressure of hydrogen;
- withdrawing, from the reactor, at least a fifth stream consisting of the reaction mixture and including hydrogen bubbles dispersed in the said mixture;
- making this fifth stream circulate in at least one loop, which runs into the bottom and the top of the reactor, and removing, by heat exchange with the said fifth stream, the heat produced by the hydrogenation reaction so as to maintain the reaction mixture at a temperature below 150°C;
- withdrawing, from this fifth stream circulating in one of the loops, a sixth stream containing a portion of the hydrogenate separated from the catalyst; and
- withdrawing, from the reactor or from one of the circulation loops, a seventh stream, of hydrogenate which is fed into a liquid/solid separation step, and recovering the liquid phase containing the catalyst-free hydrogenate and the solid phase formed by the catalyst, the said solid phase being treated in order to be regenerated before being recycled into the second, catalyst stream.

2. Process according to Claim 1, **characterized in that** the heat is removed by a heat exchanger placed downstream of the said withdrawal of the sixth stream of hydrogenated compounds in the circulation loop for the said fifth stream.

3. Process according to Claim 1 or 2, **characterized in that** an eighth stream of the reaction mixture is withdrawn from the reactor and circulated in a second loop before being recycled into the reactor, the heat being removed by a heat exchanger placed in the said circulation loop for the eighth stream.

4. Process according to one of the preceding claims, **characterized in that** the sixth stream is withdrawn through a filter medium.

5. Process according to Claim 4, **characterized in that** the filter medium is a porous medium placed tangentially to the direction of the fifth stream circulating in the loop.

6. Process according to Claim 4 or 5, **characterized in that** the porous medium is made of metal.

7. Process according to Claim 4 or 5, **characterized in that** the filter medium comprises a membrane placed on a support.

8. Process according to one of the preceding claims, **characterized in that** the reaction mixture includes a solvent.

9. Process according to one of the preceding claims, **characterized in that** the reaction mixture includes water, ammonia or an alcohol.

10. Process according to Claim 9, **characterized in that** the reaction mixture comprises from 1.5% to 7% by weight of water with respect to the liquid mass of the reaction mixture.

11. Process according to one of Claims 1 to 10, **characterized in that** the liquid/solid separation carried out on the seventh stream is a settling, filtering or centrifuging step.

12. Process according to Claim 11, **characterized in that** the liquid/solid separation is carried out in a device comprising an upper portion having a bottom in the form of a cone, and a pipe of small cross section extending from the end of the cone, a means for moving the solid being placed in the pipe of small cross section, the reaction mixture being fed into the said upper portion, the liquid phase separated from the solid being withdrawn from the said upper portion, and the settled solid being recovered from the bottom of the pipe of small cross section.

13. Process according to Claim 12, **characterized in that** water is introduced into the pipe of small cross section.

14. Process according to one of the preceding claims, **characterized in that** the solid phase recovered is subjected to a catalyst regeneration step.

15. Process according to Claim 14, **characterized in that** the regenerated catalyst is fed into the second, catalyst stream.

16. Process according to one of the preceding claims, **characterized in that** the second, catalyst stream consists of a mixture comprising the catalyst, a basic compound and optionally a solvent in which the basic compound is insoluble, the basic compound/catalyst ratio being between 0.1 mol and 50 mol of basic compounds per 1 kg of catalyst.

17. Process according to Claim 16, **characterized in that** the catalyst in the second, catalyst stream is a mixture of fresh catalyst and regenerated catalyst.

18. Process according to one of the preceding claims, **characterized in that** the catalyst is a Raney nickel or a Raney cobalt comprising between 2% and 6% by weight of aluminium and optionally a dopant chosen from chromium, titanium, molybdenum, copper, tungsten, iron, zinc, rhodium and iridium.

19. Process according to one of the preceding claims, **characterized in that** the polynitrile compound is adiponitrile.

## Patentansprüche

1. Kontinuierliches Hydrierverfahren von Verbindungen, umfassend Nitril- oder Nitrofunktionen aus Amin- oder Aminonitrilverbindungen in Gegenwart eines heterogenen Hydrierkatalysators in geteilter Form und einer basischen Verbindung, **dadurch gekennzeichnet, dass** es besteht aus dem
- Beschicken eines Rührreaktors mit;
- einem ersten Fluss von zu hydrierendem Reaktionsmittel,
- einem zweiten Fluss von Katalysator,
- einem dritten Fluss von basischer Verbindung,
- einem vierten Fluss von Wasserstoff, um den Reaktor unter Wasserstoffdruck zu halten.
- Abziehen aus dem Reaktor von mindestens einem fünften Fluss, der sich aus dem Reaktionsmedium zusammensetzt und im Medium verteilte Wasserstoffblasen umfasst,
- Zirkulieren lassen des fünften Flusses in mindestens einer Dosierschleife, die am unteren Teil und am oberen Teil des Reaktors offen ist, und Beseitigen mit diesem fünften Fluss durch Wärmeaustausch der Kalorien, die durch die Hydrierreaktion erzeugt wurden, um so das Reaktionsmedium bei einer Temperatur von unter 150 °C zu halten,
- Entnehmen aus diesem fünften Fluss, der in einer der Dosierschleifen zirkuliert, eines sechsten Flusses, der einen Teil des vom Katalysator getrennten Hydrieröls enthält,
- Entnehmen aus dem Reaktor oder aus einer der Umwälzschleifen einen siebten Fluss von Hydrieröl, der einem Flüssig-Fest-Trennschritt zugeführt wird, und Wiedererlangen der flüssigen Phase, die das Hydrieröl ohne Katalysator enthält, und der festen Phase, die sich aus dem Katalysator zusammensetzt, wobei die feste Phase zur Regenerierung behandelt wird, bevor sie in den zweiten Katalysatorfluss zurückgeführt wird.

2. Verfahren nach Anspruch 1, **dadurch gekennzeichnet, dass** die Beseitigung der Kalorien durch einen Wärmeaustauscher gewährleistet wird, der unterhalb der Entnahme des sechsten Flusses von wasserstoffhaltigen Verbindungen auf der Umwälzschleife des fünften Flusses angeordnet ist.

3. Verfahren nach Anspruch 1 oder 2, **dadurch gekennzeichnet, dass** ein achter Fluss des Reaktionsmediums aus dem Reaktor abgezogen wird und in einer zweiten Dosierschleife in Umlauf gesetzt wird, bevor er in den Reaktor zurückgeführt wird, wobei die Beseitigung von Kalorien durch einen Wärmeaustauscher gewährleistet wird, der auf der Umwälzschleife des achten Flusses angeordnet ist.

4. Verfahren nach einem der vorhergehenden Ansprüche, **dadurch gekennzeichnet, dass** die Entnahme des sechsten Flusses durch ein Filtermedium erfolgt.

5. Verfahren nach Anspruch 4, **dadurch gekennzeichnet, dass** das Filtermedium ein poröses Medium ist, das tangential zur Richtung des fünften Flusses, der in der Dosierschleife zirkuliert, angeordnet ist.

6. Verfahren nach Anspruch 4 oder 5, **dadurch gekennzeichnet, dass** das poröse Medium metallisch ist.

7. Verfahren nach Anspruch 4 oder 5, **dadurch gekennzeichnet, dass** das Filtermedium eine Membran, die auf einem Träger angeordnet ist, umfasst.

8. Verfahren nach einem der vorhergehenden Ansprüche, **dadurch gekennzeichnet, dass** das Reaktionsmedium ein Lösemittel umfasst.

9. Verfahren nach einem der vorhergehenden Ansprüche, **dadurch gekennzeichnet, dass** das Reaktionsmedium Wasser, Ammoniak oder einen Alkohol umfasst.

10. Verfahren nach Anspruch 9, **dadurch gekennzeichnet, dass** das Reaktionsmedium im Verhältnis zur flüssigen Masse des Reaktionsmediums 1,5 Gew.-% bis 7 Gew.-%. Wasser umfasst.

11. Verfahren nach einem der Ansprüche 1 bis 10, **dadurch gekennzeichnet, dass** die Flüssig-Fest-Trennung, die auf dem siebten Fluss durchgeführt wird, ein Klärungs-, Filterungs- oder Zentrifugationsschritt ist.

12. Verfahren nach Anspruch 11, **dadurch gekennzeichnet, dass** die Flüssig-Fest-Trennung in einer Vorrichtung durchgeführt wird, die einen oberen Teil, der einen Boden in Form eines Kegels aufweist, und eine Rohrleitung mit geringem Querschnitt, die sich vom Ende des Kegels aus erstreckt, umfasst, wobei ein Mittel zum Ingangbringen des Festkörpers in der Rohrleitung mit geringem Querschnitt angeordnet ist, wobei das Reaktionsmedium dem oberen Teil zugeführt wird, wobei die flüssige Phase, die vom Festkörper getrennt ist, aus dem oberen Teil entnommen wird, wobei der geklärte Festkörper aus dem unteren Teil der Rohrleitung mit geringem Querschnitt wiedererlangt wird.

13. Verfahren nach Anspruch 12, **dadurch gekennzeichnet, dass** Wasser in die Rohrleitung mit geringem Durchschnitt eingeführt wird.

14. Verfahren nach einem der vorhergehenden Ansprüche, **dadurch gekennzeichnet, dass** die wiedererlangte feste Phase einem Schritt der Regenerierung des Katalysators unterzogen wird.

15. Verfahren nach Anspruch 14, **dadurch gekennzeichnet, dass** der regenerierte Katalysator dem zweiten Katalysatorfluss zugeführt wird.

16. Verfahren nach einem der vorhergehenden Ansprüche, **dadurch gekennzeichnet, dass** sich der zweite Katalysatorfluss aus einer Mischung zusammensetzt, umfassend den Katalysator, eine basische Verbindung und gegebenenfalls ein Lösemittel, wobei die basische Verbindung nicht löslich ist, wobei das Verhältnis der basischen Verbindung zum Katalysator zwischen 0,1 Mol und 50 Mol der basischen Verbindung per 1 kg Katalysator liegt.

17. Verfahren nach Anspruch 16, **dadurch gekennzeichnet, dass** der Katalysator im zweiten Katalysatorfluss eine Mischung aus neuem Katalysator und regeneriertem Katalysator ist.

18. Verfahren nach einem der vorhergehenden Ansprüche, **dadurch gekennzeichnet, dass** der Katalysator ein Raney-Nickel oder ein Raney-Kobalt ist, umfassend zwischen 2 Gew.-% und 6 Gew.-% Aluminium und gegebenenfalls einen Dotierungsstoff, der aus Chrom, Titan, Molybdän, Kupfer, Wolfram, Eisen, Zink, Rhodium, Iridium ausgewählt ist.

19. Verfahren nach einem der vorhergehenden Ansprüche, **dadurch gekennzeichnet, dass** die Polynitrilverbindung Adiponitril ist.
